# EUROPEAN PATENT APPLICATION

(11) **EP 3 308 716 A1**
(43) Date of publication of application: **18.04.2018**
(21) Application number: 16807304.7
(22) Date of filing: 27.05.2016
(51) Int. Cl.: A61B 10/02

(54) **BIOPSY NEEDLE**

(30) Priority: 10.06.2015 JP 2015117828
(71) Applicant: Olympus Corporation, Hachioji-shi, Tokyo 192-8507 (JP)
(72) Inventor: NISHINA, Kenichi, Tokyo 192-8507 (JP); IIJIMA, Yasuhiro, Tokyo 192-8507 (JP)
(74) Representative: Schicker, Silvia
(86) International application number: PCT/JP2016/065691
(87) International publication number: WO 2016/199597

(57) **Abstract**

In a biopsy needle (100) according to the present invention, a moving mechanism is configured to cause each of an outer needle (110) and an inner needle (120) to independently slide in a longitudinal direction, and configured such that a maximum distance by which an outer needle (111) is slidable in a distal end direction is set to be longer than a maximum distance by which the inner needle (120) is slidable in the distal end direction such that, by movement of the moving mechanism, the outer needle (110) and the inner needle (120) switch between a first state, in which a second needle tip (121) of the inner needle is positioned closer to a distal end than a first needle tip (111) of the outer needle, and a second state, in which a position of the first needle tip (111) in the longitudinal direction is located closer to the distal end than a proximal end of an inclined surface (122) of the inner needle and a distance (D₂) between the first needle tip and the second needle tip is shorter than a distance (D₁) between the first needle tip and the second needle tip in the first state.

## Description

### Field

The present invention relates to a biopsy needle for collecting body tissue.

### Background

Conventionally, a biopsy is performed for pathological definitive diagnosis or the like by introducing a narrow and long biopsy needle to an observed region through a treatment tool channel of an ultrasound endoscope using an ultrasound tomogram obtained by the ultrasound endoscope as a guide, and puncturing lesion tissue to collect body tissue. As a biopsy needle, there is a proposed configuration in which an inner needle with a cutout (notch) for tissue collection formed on a side surface at a distal end side is arranged inside a hollow outer needle, and the inner needle and the outer needle are caused to advance and retract by a moving mechanism (for example, see Patent Literature 1). In the biopsy, puncture is performed with the biopsy needle to reach the tissue of a biopsy site in a state where the outer needle of the biopsy needle substantially covers the notch of the inner needle, and thereafter, the inner needle is caused to protrude from a distal end of the outer needle until the notch is exposed. Accordingly, body tissue enters the inside of the notch, and then the outer needle is caused to advance so that the body tissue that has entered the inside of the notch is cut by the distal end of the outer needle and the notch is covered by the outer needle in a state where the body tissue is caught inside the notch. In this state, the biopsy needle is pulled out from the biopsy site, and thereafter, the notch is exposed by causing the outer needle to retract, and the body tissue inside the notch is collected.

### Citation List

### Patent Literature

Patent Literature 1: JP 2009-531115 A

### Summary

### Technical Problem

A biopsy for diagnosis of prostate cancer is currently performed in a transrectal or transperineal manner, but there is a demand for a method of performing a biopsy from the inside of prostate in a transurethral manner in order to reduce invasion to a patient. With this method, it may be possible to further reduce a patient burden without causing a needle tip to contact a nerve traveling an outer side of the prostate as long as a capsule (outer membrane) of the prostate is not penetrated outwardly from the inner side.

However, in the conventional biopsy needle, the notch that enables collection of a specimen is provided in a portion at a proximal end side separated from the needle tip by about 5 mm. Therefore, if the notch is caused to reach the vicinity of a capsule boundary in order to collect tissue present in the vicinity of the capsule boundary of the prostate, there may be a case where the capsule is penetrated by the needle tip and it is difficult to deny a possibility that the needle tip contacts the nerve. Thus, there is a demand for a configuration that enables collection of tissue in the vicinity of the capsule boundary almost without causing the needle tip of the biopsy needle to move to the outside of the capsule from the capsule of the prostate.

The present invention has been made in view of the foregoing, and an object of the invention is to provide a biopsy needle capable of reliably collecting tissue and reducing a protruding length of the biopsy needle that protrudes from a collection target region in collecting the tissue.

### Solution to Problem

To solve the above-described problem and achieve the object, a biopsy needle according to the present invention includes: an outer needle having a tubular shape and including a first needle tip at one end in a longitudinal direction of the outer needle; an inner needle having a columnar shape and configured to be inserted into the outer needle movably in the outer needle in the longitudinal direction of the outer needle, the inner needle including: a second needle tip formed at a distal end of the inner needle; an inclined surface inclined toward the second needle tip at the distal end; and a notch portion comprising a notch formed on a side surface closer to a proximal end of the inner needle than the inclined surface, the notch being configured to collect body tissue; and a moving mechanism configured to cause each of the outer needle and the inner needle to independently slide in the longitudinal direction, and configured such that a maximum distance by which the outer needle is slidable in a distal end direction is set to be longer than a maximum distance by which the inner needle is slidable in the distal end direction such that, by movement of the moving mechanism, the outer needle and the inner needle switch between a first state, in which the second needle tip is positioned closer to a distal end than the first needle tip, and a second state, in which a position of the first needle tip in the longitudinal direction is located closer to the distal end than a proximal end of the inclined surface of the inner needle and a distance between the first needle tip and the second needle tip is shorter than a distance between the first needle tip and the second needle tip in the first state.

Moreover, in the biopsy needle according to the present invention, the first state is a state where a position of the first needle tip in the longitudinal direction is located closer to a proximal end than the inclined surface of the inner needle.

Moreover, in the biopsy needle according to the present invention, the first state is a state where at least a part of the notch portion is positioned inside the outer needle.

Moreover, in the biopsy needle according to the present invention, the inner needle has a cylindrical shape.

### Advantageous Effects of Invention

A biopsy needle according to the present invention includes: a moving mechanism configured to cause each of an outer needle and an inner needle to independently slide in a longitudinal direction, and configured such that a maximum distance by which the outer needle is slidable in a distal end direction is set to be longer than a maximum distance by which the inner needle is slidable in the distal end direction such that, by movement of the moving mechanism, the outer needle and the inner needle switch between a first state, in which a second needle tip is positioned closer to a distal end side than a first needle tip, and a second state, in which a position of the first needle tip in the longitudinal direction is located closer to the distal end side than a proximal end of an inclined surface of the inner needle and a distance between the first needle tip and the second needle tip is shorter than a distance between the first needle tip and the second needle tip in the first state. Therefore, it is possible to reliably collect tissue and reduce a protruding length of the biopsy needle that protrudes from a collection target region in collecting the tissue.

### Brief Description of Drawings

FIG. 1 is a cross sectional view of a biopsy needle according to an embodiment of the present invention, taken along a plane passing through a central axis in a longitudinal direction of the biopsy needle and a center of a needle tip of an inner needle.
FIG. 2 is a diagram for explaining a biopsy method implemented by the biopsy needle illustrated in FIG. 1.
FIG. 3 is a diagram for explaining a first state and a second state of the biopsy needle illustrated in FIG. 1.
FIG. 4 is a diagram for explaining protruding operations of an outer needle and the inner needle of the biopsy needle illustrated in FIG. 1.
FIG. 5A is a cross sectional view of a distal end of a conventional biopsy needle taken along a plane passing through a central axis in a longitudinal direction of the biopsy needle and a center of a needle tip of an inner needle.
FIG. 5B is a cross sectional view of a distal end of a conventional biopsy needle taken along a plane passing through a central axis in a longitudinal direction of the biopsy needle and a center of a needle tip of an inner needle.
FIG. 6A is a diagram for explaining a biopsy operation of the biopsy needle illustrated in FIG. 1.
FIG. 6B is a diagram for explaining a biopsy operation of the biopsy needle.
FIG. 6C is a diagram for explaining a biopsy operation of the biopsy needle.
FIG. 6D is a diagram for explaining a biopsy operation of the biopsy needle.
FIG. 6E is a diagram for explaining a biopsy operation of the biopsy needle.
FIG. 6F is a diagram for explaining a biopsy operation of the biopsy needle.

### Description of Embodiments

Hereinafter, modes for carrying out the present invention will be described in detail with reference to the drawings. The present invention is not limited to the embodiments below. The drawings referred to in the descriptions below schematically illustrate shapes, sizes, and positional relationships merely to make contents of the present invention understandable. That is, the present invention is not limited to only the shapes, the sizes, and the positional relationships illustrated in the individual drawings. In the following descriptions, an exemplary biopsy needle configured to puncture the interior of body tissue of an animal including a human and to collect the body tissue will be described. However, the present invention is not limited to the embodiments. The same reference signs are used to designate the same elements throughout the drawings.

### (First Embodiment)

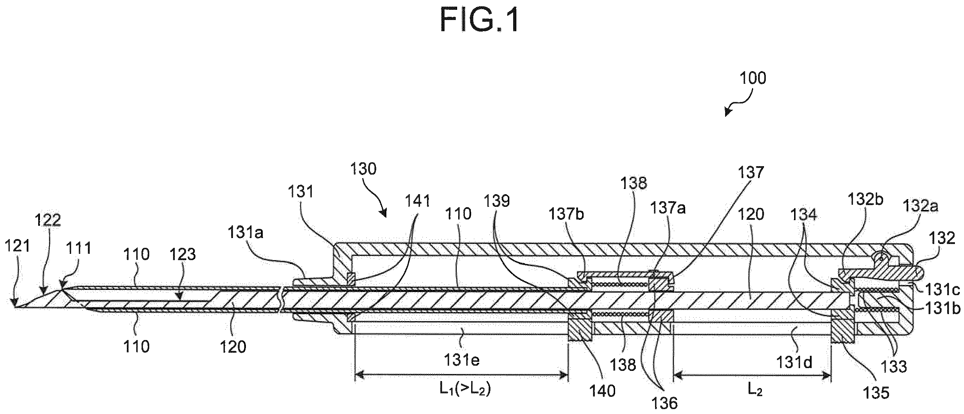

FIG. 1 is a cross sectional view of a biopsy needle according to an embodiment of the present invention, taken along a plane passing through a central axis in a longitudinal direction of the biopsy needle and a center of a needle tip of an inner needle.

As illustrated in FIG. 1, a biopsy needle 100 according to a first embodiment includes a tubular outer needle 110 extending in the longitudinal direction, a cylindrical-shaped solid inner needle 120 that is inserted into and movable in the outer needle 110 in the longitudinal direction, and an operating unit 130 that houses proximal ends of the outer needle 110 and the inner needle 120 therein and includes a moving mechanism that causes each of the outer needle 110 and the inner needle 120 to independently slide in the longitudinal direction.

The outer needle 110 has the tubular shape with a sharp distal end and includes a needle tip 111 (first needle tip) at the distal end in the longitudinal direction. The outer needle 110 made of a material having biocompatibility, for example, metal such as stainless, titanium, and aluminum or resin such as fluororesin.

The inner needle 120 includes a needle tip 121 (second needle tip) formed at a distal end, an inclined surface 122 inclined toward the needle tip 121 at the distal end, and a notch portion 123 including a notch formed on a side surface closer to a proximal end side than the inclined surface 122 to collect body tissue. The inner needle 120 made of a material having biocompatibility, similarly to the outer needle 110. The needle tip 121 is sharpened by cutting the distal end of the inner needle 120 such that an apex thereof is formed at the side opposite to the inclined surface 122 when viewed in a transverse direction. The inclined surface 122 of the distal end of the inner needle 120 is formed using a processing method such as lancet, back-cut, semi-lancet, or flat-sharpening. The inner needle 120 may have a prismatic shape instead of the cylindrical shape.

The operating unit 130 has a configuration in which a trigger button 132, an inner needle charge coil spring 133, an inner needle slider 134, an inner needle knob 135, an inner needle stopper 136, an outer needle fixing hook releasing lever 137, an outer needle charge coil spring 138, an outer needle slider 139, an outer needle knob 140, and an outer needle stopper 141, which function as the moving mechanism, are assembled with an operating unit body 131 that has a hollow columnar shape.

In the operating unit body 131, a lure pipe sleeve portion 131a is provided at a distal end, a spring assembly convex portion 131b is provided at an internal proximal end, a trigger button hole 131c is provided in an upper portion of the spring assembly convex portion 131b, an inner needle knob hole 131d dug in the longitudinal direction is provided in a bottom portion at a proximal end side, and an outer needle knob hole 131e dug in the longitudinal direction is provided in a bottom portion at the distal end side. The distal ends of the outer needle 110 and the inner needle 120 protrude in the distal end direction from the lure pipe sleeve portion 131a. A proximal end of the inner needle charge coil spring 133 to be described later is assembled with the spring assembly convex portion 131b. A proximal end of the trigger button 132 to be described later protrudes from the trigger button hole 131c. The inner needle knob 135 to be described later is slidable in the longitudinal direction inside the inner needle knob hole 131d. The outer needle knob 140 to be described later is slidable in the longitudinal direction inside the outer needle knob hole 131e.

The trigger button 132 serves as a trigger of an advancing operation of the inner needle 120, and both ends thereof move alternately up and down with a fulcrum 132a, which is connected to the inside of the operating unit body 131, as an axis. An inner needle fixing hook 132b to be caught by a concave portion of the inner needle slider 134 to be described later is provided at a distal end of the trigger button 132.

The inner needle charge coil spring 133 has the proximal end assembled with a proximal end portion of the spring assembly convex portion 131b and a distal end assembled with a side surface at a proximal end side of the inner needle slider 134 to be described later, and biases the inner needle slider 134 toward the distal end direction by being extended after compression (charge).

The inner needle slider 134 is connected to the proximal end of the inner needle 120 and advances in the distal end direction by being biased toward the distal end direction by the inner needle charge coil spring 133, and accordingly, causes the inner needle 120 to advance in the distal end direction. The concave portion to which the inner needle fixing hook 132b is fitted is formed on a top surface of the inner needle slider 134.

The inner needle knob 135 slides along the inner needle knob hole 131d so as to be able to advance and retract in the longitudinal direction. A top surface of the inner needle knob 135 is connected to a bottom surface of the inner needle slider 134. A lower portion of the inner needle knob 135 protrudes from the inner needle knob hole 131d. The inner needle knob 135 advances inside the inner needle knob hole 131d along with advancing of the inner needle slider 134. In addition, an operator of the biopsy needle 100 can retract the inner needle slider 134 and the inner needle 120 to the proximal end side by retracting the inner needle knob 135 to the proximal end side along the inner needle knob hole 131d. When the inner needle knob 135 retracts to a proximal end of the inner needle knob hole 131d, the inner needle fixing hook 132b is fitted into the concave portion of the inner needle slider 134, and the inner needle 120 is fixed at a position located at the most proximal end side.

The inner needle stopper 136 stops the advancing operation of the inner needle slider 134, and accordingly stops the advancing operation of the inner needle 120.

Both ends of the outer needle fixing hook releasing lever 137 move up and down with a fulcrum 137a, which is connected to the inner needle stopper 136, as an axis. An outer needle fixing hook 137b to be caught by a concave portion of the outer needle slider 139 to be described later is provided at a distal end of the outer needle fixing hook releasing lever 137.

The outer needle charge coil spring 138 has a proximal end assembled with a side surface at a distal end side of the inner needle stopper 136 and a distal end assembled with a side surface at a proximal end side of the outer needle slider 139 to be described later, and biases the outer needle slider 139 toward the distal end direction by being extended after compression (charge).

The outer needle slider 139 is connected to the proximal end of the outer needle 110 and advances in the distal end direction by being biased toward the distal end direction by the outer needle charge coil spring 138, and accordingly, causes the outer needle 110 to advance in the distal end direction. The concave portion to which the outer needle fixing hook 137b is fitted is formed on a top surface of the outer needle slider 139.

The outer needle knob 140 slides along the outer needle knob hole 131e so as to be able to advance and retract in the longitudinal direction. A top surface of the outer needle knob 140 is connected to a bottom surface of the outer needle slider 139. A lower portion of the outer needle knob 140 protrudes from the outer needle knob hole 131e. The outer needle knob 140 advances inside the outer needle knob hole 131e along with advancing of the outer needle slider 139. In addition, the operator of the biopsy needle 100 can retract the outer needle slider 139 and the outer needle 110 to the proximal end side by retracting the outer needle knob 140 to the proximal end side along the outer needle knob hole 131e. When the outer needle knob 140 retracts to a proximal end of the outer needle knob hole 131e, the outer needle fixing hook 137b is fitted into the concave portion of the outer needle slider 139, and the outer needle 110 is fixed at a position located at the most proximal end side.

The outer needle stopper 141 is provided at a distal end portion of the operating unit body 131, stops the advancing operation of the outer needle slider 139, and accordingly stops the advancing operation of the outer needle 110.

A length L₁ of the outer needle knob hole 131e in the longitudinal direction, that is, a stroke (maximum slidable distance) L₁ of the outer needle 110 in the distal end direction is set to be longer than a length L₂ of the inner needle knob hole 131d in the longitudinal direction, that is, the stroke L₂ of the inner needle 120 in the distal end direction. The stroke L₂ is set such that the notch portion 123 can be adequately exposed from the needle tip 111 of the outer needle 110 to the extent that the tissue collection is enabled.

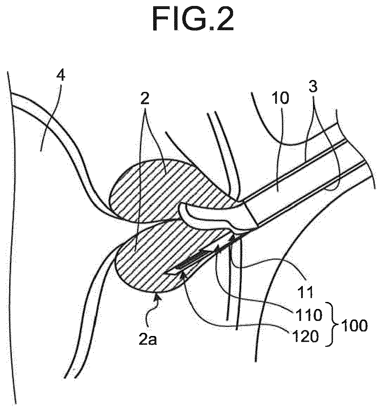

FIG. 2 is a diagram for explaining a biopsy method implemented by the biopsy needle 100. For example, as illustrated in FIG. 2, an insertion portion 10 of an ultrasound endoscope is introduced to reach a prostate 2 as a biopsy target via a urethra 3 of a patient using an ultrasound tomogram obtained by the ultrasound endoscope as a guide. A distal end of the biopsy needle 100 inserted into a treatment tool channel of the insertion portion 10 is caused to protrude from an aperture portion 11 at a distal end of the insertion portion 10 so that the outer needle 110 and the inner needle 120 puncture the prostate 2. The distal end of the inner needle 120 is caused to protrude from the outer needle 110 until the notch portion 123 is exposed, and thereafter, the outer needle 110 is caused to advance in the distal end direction so that body tissue that has entered the inside of the notch is cut by the distal end of the outer needle 110 and the notch portion 123 is covered by the outer needle 110 in a state where the body tissue is caught inside the notch. In this state, the biopsy needle 100 is pulled out of the body via the treatment tool channel, and thereafter, the body tissue caught inside the notch is collected. A bladder 4 is positioned inside the prostate 2.

In the biopsy needle 100 according to the embodiment, the notch portion 123 is extended to the distal end side of the inner needle 120, a first state and a second state to be described later are defined, and the first state and the second state are switched from one to the other in order to reduce a protruding length of the inner needle 120 such that the needle tip of the inner needle 120 does not outwardly penetrate a capsule 2a of the prostate 2 from the inner side thereof, and to enable collection of body tissue in the notch portion 123.

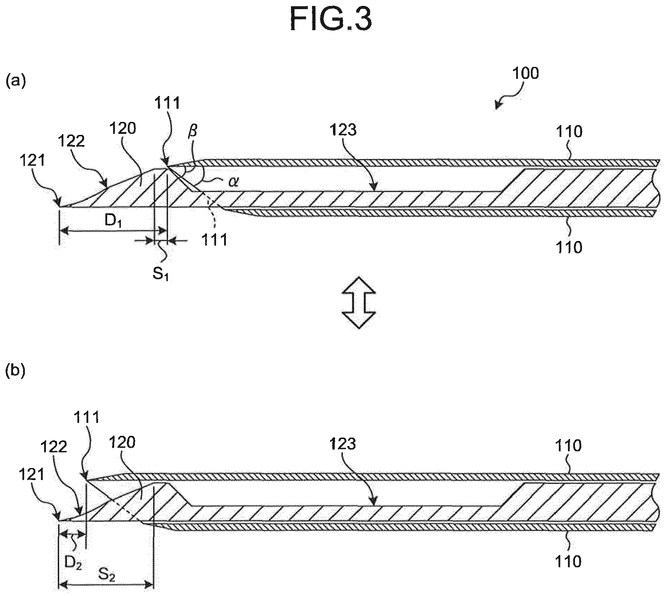

FIG. 3 is a diagram for explaining the first state and the second state of the biopsy needle 100, and is a cross sectional view of the distal end of the biopsy needle 100 taken along the plane passing through the central axis in the longitudinal direction and the center of the needle tip 121 of the inner needle 120. (a) of FIG. 3 is a diagram illustrating the first state. The first state is a charge state where both of the inner needle charge coil spring 133 and the outer needle charge coil spring 138 are compressed and charged with energy for extension. In other words, the first state is a state where the outer needle 110 and the inner needle 120 are charged with energy for protrusion, and is a state obtained when the outer needle 110 and the inner needle 120 puncture a biopsy site. As illustrated in (a) of FIG. 3, in the first state, the needle tip 121 of the inner needle 120 is positioned closer to the distal end side than the needle tip 111 of the outer needle 110. Further, as a distance between the needle tips, that is, as a distance between the needle tip 111 of the outer needle 110 and the needle tip 121 of the inner needle 120 in the longitudinal direction, a first distance D₁ is set. In addition, in the first state, it is ideal that the needle tip 111 of the outer needle 110 is positioned in a region S₁ between a proximal end of the inclined surface 122 of the inner needle 120 and a distal end of the notch portion 123. For example, the distance between the needle tip 121 of the inner needle 120 and the distal end of the notch portion 123 is narrowed to about 3 to 4 mm in the case of the biopsy needle 100 of 18 gauge. Furthermore, to increase the amount of tissue collected in the notch portion 123, an angle β of the inclined surface at the distal end of the notch portion 123 with respect to the outer tube surface of the inner needle 120 is set to be larger than an angle α of the inclined surface of the outer needle 110.

(b) of FIG. 3 is a diagram for explaining the second state of the biopsy needle 100, and is a cross sectional view of the distal end of the outer needle 110 taken along the plane passing through the central axis in the longitudinal direction and the center of the needle tip 121 of the inner needle 120. The second state is a state where the inner needle 120 and the outer needle 110 slide to the biopsy site in the distal end direction and the biopsy collection is completed. In the second state, the position of the needle tip 111 of the outer needle 110 in the longitudinal direction is located closer to the distal end side than the proximal end of the inclined surface 122 of the inner needle 120. That is, the outer needle 110 is in a state of entirely covering the notch portion 123 of the inner needle 120. In addition, in the second state, the outer needle 110 and the inner needle 120 are positioned such that a distance between the needle tip 111 of the outer needle 110 and the needle tip 121 of the inner needle 120 becomes a second distance D₂ that is shorter than the first distance D₁. Furthermore, in the second state, it is ideal that the needle tip 111 of the outer needle 110 is positioned in a region S₂ between the needle tip 121 of the inner needle 120 and the proximal end of the inclined surface 122. That is, in the second state, it is ideal that the outer needle 110 and the inner needle 120 are positioned such that the position of the needle tip 111 of the outer needle 110 in the longitudinal direction overlaps with the inclined surface 122 of the inner needle 120.

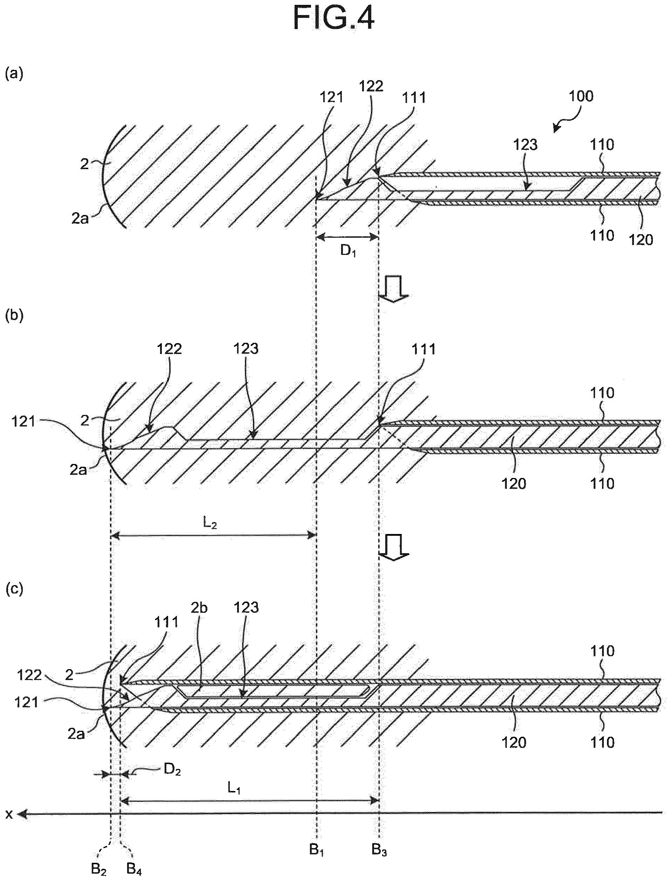

Next, FIG. 4 is a diagram for explaining protruding operations of the outer needle 110 and the inner needle 120 of the biopsy needle 100. FIG. 4 illustrates a case where the inner needle 120 and the outer needle 110 protrude in an x-axis direction.

(a) of FIG. 4 illustrates the first state (charge state) where the needle tip 121 of the inner needle 120 is positioned closer to the distal end side than the needle tip 111 of the outer needle 110, and is a state obtained before a region in the vicinity of the capsule 2a of the prostate 2 as the biopsy site is punctured.

(b) of FIG. 4 illustrates a state where only the inner needle 120 protrudes in the x-axis direction of the distal end side when the inner needle 120 is moved by the moving mechanism in order to collect tissue in the vicinity of the capsule 2a of the prostate 2. In this case, the inner needle 120 protrudes in the x-axis direction by the above-described stroke L₂ as compared to the first state. Therefore, the needle tip 121 of the inner needle 120 moves in the x-axis direction by the stroke L₂ from a position B₁ in the first state and reaches a position B₂ in the vicinity of the capsule 2a. Consequently, the notch portion 123 is fully exposed, and tissue of the prostate 2 enters the inside of the notch portion 123.

(c) of FIG. 4 illustrates a state where the outer needle 110 also protrudes and the biopsy operation is completed, that is, the second state. The outer needle 110 protrudes in the x-axis direction by the stroke L₁ as compared to the first state. The stroke L₁ is longer than the stroke L₂. Therefore, the needle tip 111 of the outer needle 110 located at a position B₃ between the proximal end of the inclined surface 122 of the inner needle 120 and the distal end of the notch portion 123 in the first state passes by the distal end of the notch portion 123 of the inner needle 120, and then reaches a position B₄ beyond the proximal end of the inclined surface 122 of the inner needle 120. In other words, after the inner needle 120 and the outer needle 110 protrude in sequence, the protruding needle tip 111 of the outer needle 110 reaches the vicinity of the needle tip 121 of the inner needle 120. Therefore, the distance between the needle tip 111 of the outer needle 110 and the needle tip 121 of the inner needle 120 becomes the second distance D₂ (< D₁). With the protrusion of the outer needle 110, it is possible to cut the tissue of the prostate 2, which has entered the inside of the notch portion 123, by the needle tip 111 of the outer needle 110, and entirely cover the notch portion 123 by the outer needle 110 in a state where body tissue 2b is caught inside the notch portion 123.

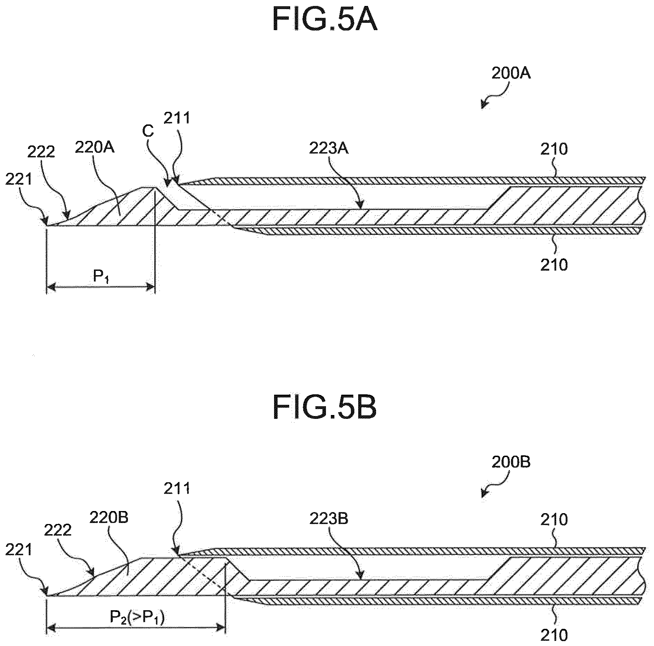

Next, conventional biopsy needles will be described. FIG. 5A and FIG. 5B are cross sectional views of distal ends of conventional biopsy needles 200A and 200B taken along a plane passing through central axes in a longitudinal direction of the biopsy needles 200A and 200B and centers of needle tips 221 of inner needles 220A and 220B. In a configuration in which a notch portion 223A is extended in a distal end direction to the vicinity of a proximal end of an inclined surface 222 and a length P₁ of the inner needle 220A protruding from a collection target region in tissue is reduced to about 2 to 3 mm as in the biopsy needle 200A illustrated in FIG. 5A in order to perform tissue collection without deeply inserting the needle into the tissue, a large gap C may be generated between a needle tip 211 of an outer needle 210 and the notch portion 223A even when the outer needle 210 protrudes, due to manufacturing variation, temperature change, or the like. In this case, it is difficult to cut body tissue by the needle tip 211 of the outer needle 210, and it is difficult to collect an adequate amount of tissue because tissue escapes from the gap C when the biopsy needle 200A is pulled out. Therefore, conventionally, it has been necessary to increase a length P₂ from the needle tip 221 of the inner needle 220B to a notch portion 223B to about 5 to 6 mm to set a certain margin so that even a distal end of the notch portion 223B can be substantially covered after the outer needle 210 protrudes as in the biopsy needle 200B illustrated in FIG. 5B. Thus, in the biopsy needle 200B, the needle tip 221 of the inner needle 220 needs to deeply puncture to a depth deeper than a region where tissue to be collected is present, as compared to the biopsy needle 200A.

In contrast, the biopsy needle 100 according to the embodiment is configured such that the notch portion 123 of the inner needle 120 is extended to the vicinity of the proximal end of the inclined surface 122 to reduce the length of the inner needle 120 protruding from a collection target region in tissue, to thereby enable tissue collection without deeply inserting the needle into the tissue. In addition, the biopsy needle 100 is configured such that the stroke (sliding distance) L₁ of the outer needle 110 in the longitudinal direction is set to be longer than the stroke L₂ of the inner needle 120 in the longitudinal direction in order to enable switching from the first state, which is a charge state where the needle tip 121 of the inner needle 120 is positioned closer to the distal end side than the needle tip 111 of the outer needle 110, to the second state, which is a state where the inner needle 120 and the outer needle 110 slide in the distal end direction and the position of the needle tip 111 of the outer needle 110 in the longitudinal direction is located closer to the distal end side than the proximal end of the inclined surface 122 of the inner needle 120, such that a gap is not generated between the needle tip 111 of the outer needle 110 and the notch portion 123 even when the outer needle 110 protrudes. That is, a configuration is realized that enables switching from the first state to the second state where the outer needle 110 and the inner needle 120 are positioned such that the distance between the needle tip 111 and the needle tip 121 becomes the second distance D₂ shorter than the first distance D₁ in the first state, and a gap is not generated between the needle tip 111 of the outer needle 110 and the notch portion 123 even when the outer needle 110 protrudes. Therefore, according to the biopsy needle 100, it is possible to reliably cut body tissue by the needle tip 111 of the outer needle 110, and secure the tissue inside the notch portion 123 of the inner needle 120, so that it is possible to collect an adequate amount of tissue.

Meanwhile, the first state is a state before collection of body tissue, and therefore, the needle tip 111 of the outer needle 110 does not necessarily have to be positioned in a place overlapping with the inclined surface 122. In other words, in the first state, the needle tip of the outer needle 110 does not necessarily have to reach the distal end of the notch portion 123, but it is sufficient that at least a part of the notch portion 123 is positioned inside the outer needle 110 and it is acceptable that a gap is generated between the needle tip 111 of the outer needle 110 and the notch portion 123. Furthermore, in the second state, it is possible to reliably cut tissue and secure the cut tissue inside the notch portion 123 as long as the outer needle 110 can entirely cover the notch portion 123. Therefore, it is sufficient that the position of the needle tip 111 of the outer needle 110 in the longitudinal direction is located closer to the distal end side than the proximal end of the inclined surface 122 of the inner needle 120, and it is not necessary that the position of the needle tip 111 of the outer needle 110 in the longitudinal direction overlaps with the inclined surface 122 of the inner needle 120.

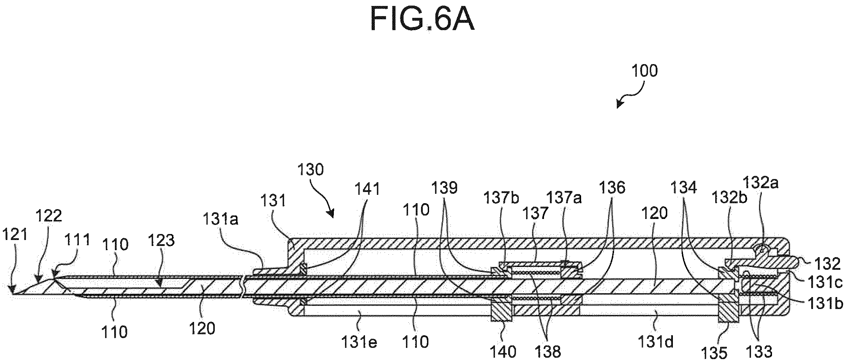

Next, a biopsy operation of the biopsy needle will be described in detail. FIG. 6A to FIG. 6F are diagrams for explaining the biopsy operation of the biopsy needle, and are cross sectional views of the distal end of the biopsy needle 100 taken along a plane passing through the central axis in the longitudinal direction of the biopsy needle 100 and the center of the needle tip of the inner needle 120.

FIG. 6A is a diagram illustrating the first state of the biopsy needle 100 described above. As illustrated in FIG. 6A, in the first state, the inner needle fixing hook 132b is caught by the concave portion of the inner needle slider 134, the outer needle fixing hook 137b is caught by the concave portion of the outer needle slider 139, and both of the inner needle charge coil spring 133 and the outer needle charge coil spring 138 are compressed and charged with the energy for extension.

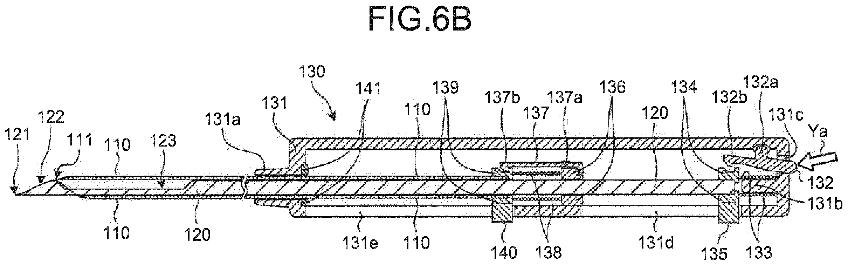

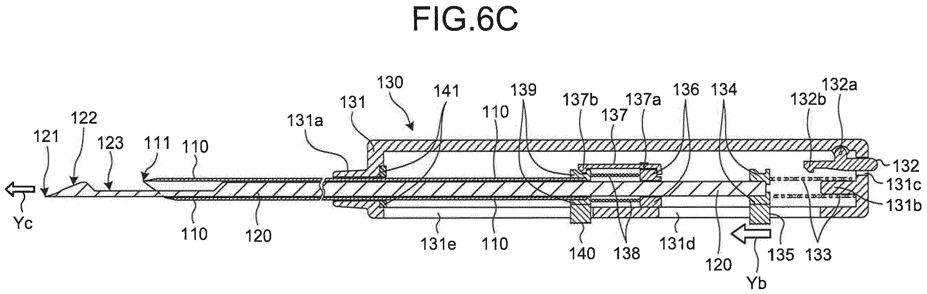

When the trigger button 132 is pressed as indicated by an arrow Ya in FIG. 6B, the inner needle fixing hook 132b at the distal end portion moves up with the fulcrum 132a as an axis and is released from the concave portion of the inner needle slider 134, the inner needle charge coil spring 133 that has been compressed extends in the distal end direction as illustrated in FIG. 6C, and the inner needle slider 134 is biased due to the extension of the inner needle charge coil spring 133 and slides in the distal end direction as indicated by an arrow Yb. Accordingly, the inner needle 120 also slides in the distal end direction as indicated by an arrow Yc.

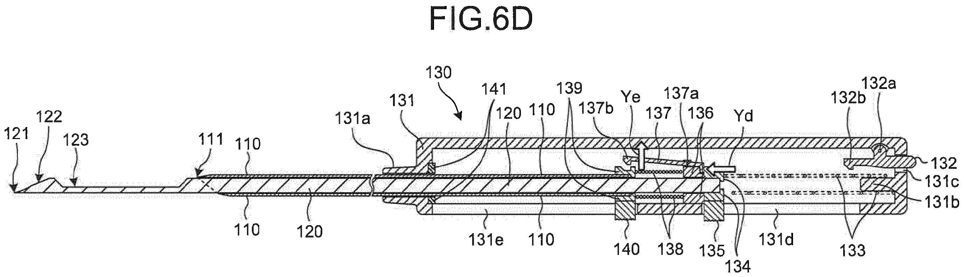

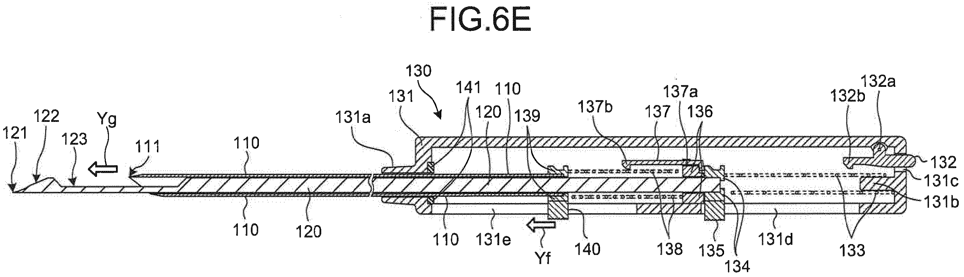

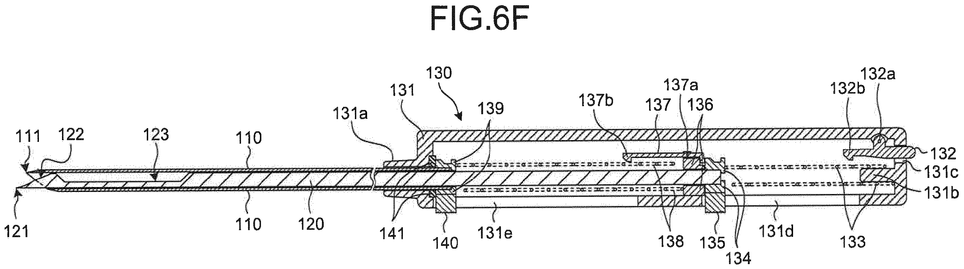

Further, as illustrated in FIG. 6D, the inner needle slider 134 and the inner needle knob 135 slide in the distal end direction until coming in contact with the inner needle stopper 136 due to the biasing of the inner needle charge coil spring 133. Accordingly, the inner needle 120 protrudes in the x-axis direction by the stroke L₂. Furthermore, the inner needle slider 134 presses the outer needle fixing hook releasing lever 137 from the proximal end side as indicated by an arrow Yd due to the biasing of the inner needle charge coil spring 133, so that the outer needle fixing hook 137b at the distal end portion moves up with the fulcrum 137a as an axis as indicated by an arrow Ye and is released from the concave portion of the outer needle slider 139. Accordingly, the outer needle charge coil spring 138 that has been compressed extends in the distal end direction as illustrated in FIG. 6E, and the outer needle slider 139 and the outer needle knob 140 move in the distal end direction until coming in contact with the outer needle stopper 141 as indicated by an arrow Yf by being biased by the extension of the outer needle charge coil spring 138. Consequently, the outer needle 110 protrudes in the x-axis direction by the stroke L₁ as indicated by an arrow Yg while cutting the tissue that has entered the notch portion 123 using the needle tip 111, and thus the biopsy needle 100 is switched to the second state as illustrated in FIG. 6F.

The operator of the biopsy needle 100 pulls out the biopsy needle 100 from the biopsy site in this state. Then, the outer needle 110 is retracted (see FIG. 6D) by moving the outer needle knob 140 to the proximal end of the outer needle knob hole 131e to expose the notch portion 123, and the body tissue inside the notch portion 123 is collected. Thereafter, the biopsy needle 100 is switched to the first state by moving the inner needle knob 135 to the proximal end of the inner needle knob hole 131d (see FIG. 6A).

In this manner, the biopsy needle 100 includes the moving mechanism configured such that the stroke L₁ of the outer needle 110 in the longitudinal direction is set to be longer than the stroke L₂ of the inner needle 120 in the longitudinal direction, and configured to cause each of the outer needle 110 and the inner needle 120 to independently move in a slidable manner in the longitudinal direction, to thereby enable switching between the first state and the second state. The moving mechanism that causes each of the outer needle and the inner needle to independently slide in the longitudinal direction is configured such that a maximum distance (the stroke L₁) by which the outer needle 110 is slidable in the distal end direction is set to be longer than a maximum distance (the stroke L₂) by which the inner needle 120 is slidable in the distal end direction such that, by movement of the moving mechanism, the outer needle 110 and the inner needle 120 switch between the first state, in which the needle tip 121 is positioned closer to the distal end side than the needle tip 111, and the second state, in which the position of the needle tip 111 in the longitudinal direction is located closer to the distal end side than the proximal end of the inclined surface 122 of the inner needle 121 and a distance between the needle tips is shorter than a distance between the needle tips in the first state.

While an example has been described in the embodiment in which the tissue of the prostate 2 is collected using the biopsy needle 100, it is of course possible to use the disclosed technology to collect tissue in other regions. In addition, while an example has been described in the embodiment in which the distal end of the biopsy needle 100 is caused to reach the biopsy site through the treatment tool channel of the insertion portion 10 of the ultrasound endoscope, the biopsy needle 100 may be inserted from the outside of a body without passing through the treatment tool channel of the ultrasound endoscope, depending on biopsy sites.

### Industrial Applicability

As described above, the biopsy needle according to the present invention is useful for reliably collecting tissue and reducing the protruding length of the biopsy needle that protrudes from a collection target region in collecting the tissue.

### Reference Signs List

- 2: PROSTATE
- 2a: CAPSULE
- 3: URETHRA
- 4: BLADDER
- 10: INSERTION PORTION
- 11: APERTURE PORTION
- 100, 200A, 200B: BIOPSY NEEDLE
- 110, 210: OUTER NEEDLE
- 111, 121, 211, 221: NEEDLE TIP
- 120, 220A, 220B: INNER NEEDLE
- 122, 222: INCLINED SURFACE
- 123, 223A, 223B: NOTCH PORTION
- 130: OPERATING UNIT
- 131: OPERATING UNIT BODY
- 131a: LURE PIPE SLEEVE PORTION
- 131b: SPRING ASSEMBLY CONVEX PORTION
- 131c: TRIGGER BUTTON HOLE
- 131d: INNER NEEDLE KNOB HOLE
- 131e: OUTER NEEDLE KNOB HOLE
- 132: TRIGGER BUTTON
- 132a, 137a: FULCRUM
- 132b: INNER NEEDLE FIXING HOOK
- 133: INNER NEEDLE CHARGE COIL SPRING
- 134: INNER NEEDLE SLIDER
- 135: INNER NEEDLE KNOB
- 136: INNER NEEDLE STOPPER
- 137: OUTER NEEDLE FIXING HOOK RELEASING LEVER
- 137b: OUTER NEEDLE FIXING HOOK
- 138: OUTER NEEDLE CHARGE COIL SPRING
- 139: OUTER NEEDLE SLIDER
- 140: OUTER NEEDLE KNOB
- 141: OUTER NEEDLE STOPPER

## Claims

1. A biopsy needle comprising:
an outer needle having a tubular shape and including a first needle tip at one end in a longitudinal direction of the outer needle;
an inner needle having a columnar shape and configured to be inserted into the outer needle movably in the outer needle in the longitudinal direction of the outer needle, the inner needle including:
a second needle tip formed at a distal end of the inner needle;
an inclined surface inclined toward the second needle tip at the distal end; and
a notch portion comprising a notch formed on a side surface closer to a proximal end of the inner needle than the inclined surface, the notch being configured to collect body tissue; and
a moving mechanism
configured to cause each of the outer needle and the inner needle to independently slide in the longitudinal direction, and
configured such that a maximum distance by which the outer needle is slidable in a distal end direction is set to be longer than a maximum distance by which the inner needle is slidable in the distal end direction such that, by movement of the moving mechanism, the outer needle and the inner needle switch between a first state, in which the second needle tip is positioned closer to a distal end than the first needle tip, and a second state, in which a position of the first needle tip in the longitudinal direction is located closer to the distal end than a proximal end of the inclined surface of the inner needle and a distance between the first needle tip and the second needle tip is shorter than a distance between the first needle tip and the second needle tip in the first state.

2. The biopsy needle according to claim 1, wherein the first state is a state where a position of the first needle tip in the longitudinal direction is located closer to a proximal end than the inclined surface of the inner needle.

3. The biopsy needle according to claim 1, wherein the first state is a state where at least a part of the notch portion is positioned inside the outer needle.

4. The biopsy needle according to claim 1, wherein the inner needle has a cylindrical shape.
